# EUROPEAN PATENT SPECIFICATION

(11) **EP 1 778 258 B1**
(45) Date of publication and mention of the grant of the patent: **07.01.2009**
(21) Application number: 05778952.1
(22) Date of filing: 28.07.2005
(51) Int. Cl.: A61K 35/74, A61P 17/00

(54) **USE OF PROBIOTIC BACTERIA FOR THE PREPARATION OF TOPICAL COMPOSITIONS FOR SKIN PROTECTION**
VERWENDUNG VON PROBIOTISCHEN BAKTERIEN ZUR HERSTELLUNG VON TOPISCHEN ZUSAMMENSETZUNGEN ZUM SCHUTZ DER HAUT
UTILISATION DE BACTERIES PROBIOTIQUES DANS LA PREPARATION DE COMPOSITIONS TOPIQUES DESTINEES A LA PROTECTION DE LA PEAU

(30) Priority: 29.07.2004 IT MI20041550
(43) Date of publication of application: 02.05.2007
(73) Proprietor: Proge Farm S.r.l., 28100 Novara (IT)
(72) Inventor: DONDI, Giancarla, I-28100 Novara (IT); MALFA, Patrizia, I-28100 Novara (IT)
(74) Representative: Trupiano, Federica
(86) International application number: PCT/IB2005/002235
(87) International publication number: WO 2006/013441

(56) References cited:
- US-A1- 2003 049 231
- US-B1- 6 258 355
- ROSENFELDT V ET AL: "EFFECT OF PROBIOTIC LACTOBACILLUS STRAINS IN CHILDREN WITH ATOPIC DERMATITIS" February 2003 (2003-02), JOURNAL OF ALLERGY AND CLINICAL IMMUNOLOGY, MOSBY - YEARLY BOOK, INC, US, PAGE(S) 389-395 , XP008023585 ISSN: 0091-6749 the whole document

## Description

### Field of the invention

The present invention concerns the use of some probiotical bacteria in the protection of the skin. More particularly, subject of the invention is the use of some probiotical bacteria in the preparation of topical compositions for the treatment of the skin diseases caused by pathogens, especially recidivant infections or dermatitis-like diseases.

The treatment of the cutaneous diseases caused by the proliferation of pathogens is conventionally carried out with antimicrobic medicaments, namely with pharmaceutical molecules with antibiotical and/or antimycotic action.

### Background of the invention

However it has been demonstrated that some particular conditions, such as the protracted treatment with antimicrobic medicaments, the unthought use of aggressive agents, such as detergents, the excessive sweating or also environmental conditions such as warmth and high moistness that increase the sweating of the person, can lead to unbalances promoting the proliferation of opportunistic pathogens.

In spite of the above mentioned problems, in the last years the greater part of the scientific efforts has been directed to the search of new antibiotic and/or antimycotic molecules more and more effective.

Searches have led to an improvement of the treatment of acute infections, but enhanced a still unsolved and very serious problem, namely the occurence of antibiotic-resistance with consequent reappearing of the infectious event (Elmer G.W. et al., JAMA, 275:870-876, 1996). These phenomena have been disclosed particularly in the infections of the feminine urogenital apparatus (Sparks J.M., J. Reprod. Med., 36: 745-752, 1991) and in the skin.

Among the various infectious diseases of the skin that show the occurence of recidives, *Pityriasis versicolor* is a very frequent infection.

This infection, caused by the fungus *Malassezia furfur,* very diffused among the youngest population, is not a serious pathology, but very bothering and generally shows a cyclic reappearing.

Some studies also show that *M. furfur* yeast plays an important role either in the seborrheic dermatitis (Bergbrant I.M. e Faergemann J., Mycoses, 37(5-6):217-219, 1994) and in the atopic dermatitis (Broberg A. e Faergemann J., Acta Derm Venereol., 75(1):46-49, 1995). The mechanism of action of the yeast in these pathologies has not yet been cleared, but there seems to be a connection with the reduction of the lymphocytic function (Faergemann J., Mycoses, 40 (suppl.1): 29-32, 1997).

Nowadays there are no *in vitro* nor *in vivo* studies that demonstrate the efficacy of probiotical bacteria at the cutaneous level and moreover many uncertainties remain on the composition and the organisation of the microbic ecosystem.

For example, Ouwehand A.C. et al. (Letters in Applied Microbiology, 2003, 36:327-331) have recently published a study carried out on different probiotical bacteria with the aim of determining their eventual benefits on the skin. The authors have estimated the capability of some probiotics of inhibiting the bacterial *in vitro* growth of various pathogens and of modifying their adhesion on the immobilized keratine. Even if a sufficient *in vitro* inhibition of the pathogens by the probiotics used has been noticed, the disappointing essay results on keratine brought the authors to the conclusion that said probiotics are not able to prevent the adhesion of said pathogens (page 331, first column, first paragraph).

### Summary of the invention

Contrary to these teachings, it was surprisingly found that, when applied on the skin, some probiotical bacteria produce a beneficial effect, opposing the infection by the pathogens.

Object of the present invention is to provide a new treatment for the affections of the skin caused by pathogens that does not exhibit the drawbacks of the prior art.

More particularly, object of the invention is to provide a treatment for the infections of the skin that is effective without risks for the patient and that reduces the occurence of recidives.

In fact it was surprisingly found that it is possible to treat microbic diseases of the skin with some probiotical bacteria obtaining in addition to the resolution of the infectious event, also the reduction of the risk of the occurence of recidives.

### Detailed description of the Invention

Therefore, according to one of its aspects, subject of the present invention is the use of specific probiotical bacteria for the preparation of a topical composition for the protection of the skin from the microbical diseases, in particular for preventing and/or treating the microbical diseases of the skin.

According to another of its aspects, subject of the invention is the use of specific probiotical bacteria for the preparation of a topical composition for preventing and/or treating the *Pityriasis versicolor.*

According to a further aspect, subject of the invention is the use of specific probiotical bacteria for the preparation of a topical composition for preventing and/or treating dermatitis, in particular the atopic dermatitis.

According to another of its aspects, subject of the invention is the use of specific probiotical bacteria for preventing and/or treating dandruff.

As mentioned here, the term "probiotical bacteria" (or also only "probiotics") indicates bacteria able to have a beneficial effect on the host organism if taken in due amounts (Guarner F., Schaafsma G.J., Int. J. Food Microbiol. 39:237-238, 1998). Among the probiotical bacteria, the ones according to the invention are those belonging to the genus *lactobacillus* and in particular *L*. *plantarum* BCCM-LMG P 17630 commercialized under trademark Bactocin® and those disclosed in Patent Application EP 0 949 330 in the name of the same

Applicant, namely *L.gasseri* BCCM-LMG P 18137, *L.gasseri* BCCM-LMG P17632 and the *L. crispatus* BCCM-LMG P 17631.

It has been demonstrated that in particular *L. crispatus* P17631 is highly effective in the treatment and in the prevention of the *Pityriasis versicolor,* also of the atopic dermatitis and its use in the preparation of products intended to such pathologies represents a preferred aspect of the present invention.

According to a preferred embodiment, the probiotical bacteria are used according to the invention in lyophilized form. The freeze-drying can be carried out according to any appropriate method, well known by those skilled in the art, optionally by using conventionally employed excipients.

The probiotical bacteria according to the invention can be included in pharmaceutical or cosmetic compositions for topical use either ready for use or for extemporaneous formulations.

Possible common additives and stabilisers can be added to said compositions or to the probiotical bacteria during the freeze-drying phase.

Thus, according to another of its aspects, subject of the invention is the use of the said probiotical bacteria in lyophilized form for the preparation of topical compositions for the prevention and/or the treatment of the microbical diseases of the skin.

For example, if desired or necessary, in case of the treatment of the dandruff, the probiotical bacteria according to the invention can be used under powder lyophilized form without being further manufactured o included in particular formulations.

According to an embodiment, subject of the invention is the use of *L*. *crispatus* P17631, advantageously in lyophilized form, for the preparation of a medicament for preventing and/or treating the infections caused by *Pityriasis versicolor.*

Preferably, the probiotical bacteria are in lyophilized form and are contained in pharmeceutical compositions for topical use, optionally in presence of conventional excipients and stabilisers.

The topical compositions comprising the said probiotical bacteria are a further subject of the invention.

Such compositions can be medicaments or cosmetic products and can be prepared according to the methods known in the art and can be in a dosage unit form or a multiple dosage form.

For illustrative purpose, the compositions of the invention can be prepared under the form of gel, creams, emulsions, ointments, foams, powders, aqueous solutions or suspensions, oil solutions or suspensions or also biphasic solutions or suspensions, to be stirred before use.

Compositions can contain between 10³ and 10¹², advantageously between 10⁵ and 10¹⁰, for example between 10⁶ and 10⁸ CFU (colony-forming units) per gram of composition.

Such compositions could be admnistered in appropriate amounts to the extent of the area to be treated, preferably one or more times a day.

The treatment is preferably extended during at least 7-10 days, however also shorter treatments can provide interesting results. Moreover, considering the absence of toxicity of the compositions of the invention, if desired or necessary longer treatments can be obviously foreseen.

It has been also observed that in some cases it is advisable to carry out a "preventing" treatment in particular periods of the year, especially in the so-called "changes of season", namely in spring and autumn.

The use of the probiotical bacteria according to the invention can also be carried out in connection with a conventional initial antimicrobic treatment, in order to accelerate the disappearing of the symptomatology (quick effect of the antimicrobic) and subsequency to limit the possibilities of relapses (long lasting effect of the probiotical bacteria).

For the use according to the invention a single probiotical bacterium or probiotical bacteria mixtures can be used indifferently. According to another aspect, the invention concerns a use for the treatment of the skin diseases as described above that comprises the topical administration of an effective dose of at least one of the said probiotical bacteria.

As previously described, the types of the dermatological preparations that maintain a satisfactory vitality of the probiotics must contain a little water and must be lypophilic as for example the following:
- Formulations wherein the medium is formed by a fluid lypophilic phase (oil) in association with a solid colloid in order to form a system with a semi-solid consistency
- Formulations formed by one lypophilic substance in association with detergents;
- Formulations formed by a lypophilic substance added with different synthesis polymers able to form a semi-solid system with flexible-viscous features adapted for the topical use (lypophilic gel).

According to an embodiment, it has been demonstrated that a biphasic liquid system (emulsions, nanoemulsions, microemulsions) such as oil in water (O/A) gives good results on the survival of the probiotics, specially if the two phases are a mixed just before use (extemporaneous formulations). In this case, the lypophilic and the hydrophilic phase are contained in two separate containers that by their opening before use allow the mixing of the two phases resulting in a mixture (squeezable) that has to be blended for example on the palm of the hand before the topical administration.

The following examples are provided in an illustrative but non limiting way.

### Example 1: Formulation of a standard ointment that comprises at least:

Lyophilized lactobacillus
Vaseline Oil
Mineral Oil
Mixture of Fatty acids with Polyethylene glycol

### Example 2: Formulation of a standard foam that comprises at least:

Lyophilized lactobacillus
Vaseline Oil
Surfactants (for example sorbitan esters)

### Example 3: Formulation of a standard gel that comprises at least

Lyophilized lactobacillus
Mixture of styrene polymers
Mineral Oil
Colloidal silica

Other components and eccipients can be obviously added to the formulations described above according to the techniques well known by those skilled in the art (for example jojoba oil, natural extracts, vegetal extracts, etc).

The activity of the probiotical bacteria according to the invention has been demonstrated with *in vitro* and *in vivo* studies.

### In Vitro Essay

*In vitro* essays has been carried out in order to estimate the antimycotic activity of the probiotical bacteria towards the growth of the pathogen *M. furfur* by changing the conditions of development in such way that they were optimal for both microorganisms (anaerobiosis + aerobiosis). Such conditions do not correspond to the growth conditions of the greater part of the probiotical bacteria, for example the Lactobacillus that prefer anaerobic environments, but nevertheless e in a surprisingly way, the results of the *in vivo* essays have demonstrated that such probiotical bacteria have in any case the desired therapeutical (or cosmetic) effect.

The *in vitro* test used is the "agar spot test" described in many articles among which the one of Jacobsen C.N. ET to. (Appl. and Envir. Microb. 65: 4949-4956, 1999) and of Ouwehand A.C. ET to. (Letters in Applied Microbiology, 2003, 36:327-331).

Spots of lactobacilli to be evaluated for their antimycotic activity were put on Petri dishes containing 15 mililiter of MRS agar, with 10µl of the culture grown for one night at 37°C. The dried dishes, left to dry for approximately 15 minutes, were incubated at 37°C for one night in anaerobic conditions.

The day after, after having checked the growth of the spots, on each dish 2,5 ml of soft agar medium mYPG were speeded, which composition is quoted in the table, and 500µl of *Malassenzia furfur* culture were added.

**Table: Composition of the soft agar medium mYPG**

| | |
|---|---|
| Malt Extract | 5g |
| Peptone | 10g |
| Glucose | 20g |
| Tween 40 | 1g |
| Tween 80 | 1g |
| Agar | 4,5g |
| Distilled water | 1000 mL |

The dishes have been incubated at 37°C under aerobic conditions for 24-48 hours, until the patina growth of the pathogen was visible.

Inhibiting areolas, more or less large, were noticed, near to the spots, where the lactobacillus sample has been seeded, bigger are the areolas diameter, more important is the antimycotic activity.

### In vivo essay

*In vivo* tests have been carried out on volunteers that every year showed recidived of *Pityriasis versicolor.*

The patients, annually, showed small white spots on the back and on the arms, due to the distruction of the melanocytes.

The treatment has been performend by applying the lyophilized of *L. crispatus* extemporaneously suspended in an oleous mixture.

The results of this essay have been surprising, because only a few administration of *L. crispatus* have caused the disappearance of the typical spots of the mycosis.

It has been possible to notice that where the suspension of *L. crispatus* was not applied the lesions were still present, further, after one year, the spots did not reappear in the treated areas.

## Claims

1. Use of at least one probiotical bacterium selected from L. plantarum, P17630, L. crispatus P17631, L. gasseri P17632, L. gasseri P18137 and their mixtures for the preparation of a topical composition for preventing and/or treating skin diseases.

2. Use according to claim 1, for the preparation of a topical composition for preventing and/or treating microbical affections of the skin.

3. Use according to claim 1, for the preparation of a topical composition for preventing and/or treating atopic dermatitis.

4. Use according to claim 2, for the preparation of a topical composition for preventing and or treating of Pityriasis versicolor.

5. Use according to claim 1, for preventing and/or treating dandruff.

6. Use according to claim s 1 to 5, wherein said probiotical bacterium is L. crispatus P17631.

7. Topical composition for use in preventing and/or treating skin diseases comprising at least one probiotical bacterium selected from L. plantarum, P17630, L. crispatus P17631, L. gasseri P17632, L. gasseri P18137 and their mixtures.

8. Composition according to claim 7, comprising from 10³ to 10¹² of CFU probiotical bacteria per gram of composition.

9. Composition according to claim 8, comprising from 10⁶ to 10⁸ of CFU probiotical bacteria per gram of composition.

10. Composition according to claims from 7 to 9, under the form of gel, cream, emulsion, ointment, foam, powder, aqueous solution or suspension, oil solution or suspension or biphasic solution or suspension.

11. Composition according to claims 7 to 10 as a single dosage unit form or as a multiple dosage unit form.

12. Composition according to claims from 7 to 11, which is a medicament or a cosmetic.

13. Composition according to claims from 7 to 12, wherein said at least one probiotical bacterium is L. crispatus P17631.

14. Composition according to claims from 7 to 12, wherein said at least one probiotical bacterium is L. plantarum, P17630.

## Patentansprüche

1. Verwendung wenigstens eines probiotischen Bakteriums, ausgewählt aus L. plantarum P17630, L. crispatus P17631, L. gasseri P17632, L. gasseri P18137 und deren Mischungen zur Herstellung einer topischen Zusammensetzung für die Prävention und/oder Behandlung von Hautkrankheiten.

2. Verwendung nach Anspruch 1 zur Herstellung einer topischen Zusammensetzung für die Prävention und/oder Behandlung von Mikrobenbefall der Haut.

3. Verwendung nach Anspruch 1 zur Herstellung einer topischen Zusammensetzung für die Prävention und/oder Behandlung von atopischer Dermatitis.

4. Verwendung nach Anspruch 2 zur Herstellung einer topischen Zusammensetzung für die Prävention und/oder Behandlung von Pityriasis versicolor.

5. Verwendung nach Anspruch 1 zur Prävention und/oder Behandlung von Kopfschuppen.

6. Verwendung nach den Ansprüchen 1 bis 5, wobei das probiotische Bakterium L. crispatus P17631 ist.

7. Topische Zusammensetzung zur Verwendung bei der Prävention und/oder Behandlung von Hautkrankheiten, umfassend wenigstens ein probiotisches Bakterium, das ausgewählt ist aus L. plantarum P17630, L. crispatus P17631, L. gasseri P17632, L. gasseri P18137 und Mischungen derselben.

8. Zusammensetzung nach Anspruch 7, umfassend 10³ bis 10¹² CFU probiotische Bakterien pro Gramm Zusammensetzung.

9. Zusammensetzung nach Anspruch 8, umfassend 10⁶ bis 10⁸ CFU probiotische Bakterien pro Gramm Zusammensetzung.

10. Zusammensetzung nach den Ansprüchen 7 bis 9 in Form eines Gels, einer Creme, Emulsion, Salbe, eines Schaums, Pulvers, einer wässrigen Lösung oder Suspension, Öllösung oder -suspension oder zweiphasigen Lösung oder Suspension.

11. Zusammensetzung nach den Ansprüchen 7 bis 10 in Form einer Einzeldosiereinheit oder in Form einer Mehrfachdosiereinheit.

12. Zusammensetzung nach den Ansprüchen 7 bis 11, bei der es sich um ein Medikament oder ein Kosmetikum handelt.

13. Zusammensetzung nach den Ansprüchen 7 bis 12, wobei das wenigstens eine probiotische Bakterium L. crispatus P17631 ist.

14. Zusammensetzung nach den Ansprüchen 7 bis 12, wobei das wenigstens eine probiotische Bakterium L. plantarum P17630 ist.

## Revendications

1. Utilisation d'au moins une bactérie probiotique choisie parmi L. plantarum, P17630, L. crispatus P17631, L. gasseri P17632, L. gasseri P18137 et leurs mélanges pour la préparation d'une composition topique destinée à la prévention et/ou au traitement de maladies cutanées.

2. Utilisation selon la revendication 1, pour la préparation d'une composition topique destinée à la prévention et/ou au traitement d'affections microbiennes de la peau.

3. Utilisation selon la revendication 1, pour la préparation d'une composition topique destinée à la prévention et/ou au traitement de la dermatite atopique.

4. Utilisation selon la revendication 2, pour la préparation d'une composition topique destinée à la prévention et/ou au traitement du Pityriasis versicolor.

5. Utilisation selon la revendication 1, pour la prévention et/ou le traitement des pellicules.

6. Utilisation selon les revendications 1 à 5, dans laquelle ladite bactérie probiotique est L. crispatus P17631.

7. Composition topique utilisable pour la prévention et/ou le traitement de maladies cutanées comprenant au moins une bactérie probiotique choisie parmi L. plantarum, P17630, L. crispatus P17631, L. gasseri P17632, L. gasseri P18137 et leurs mélanges.

8. Composition selon la revendication 7, comprenant 10³ à 10¹² bactéries probiotiques souches par gramme de composition.

9. Composition selon la revendication 8, comprenant 10⁶ à 10⁸ bactéries probiotiques souches par gramme de composition.

10. Composition selon les revendications 7 à 9, sous la forme d'un gel, d'une crème, d'une émulsion, d'un onguent, d'une mousse, d'une poudre, d'une solution ou suspension aqueuse, d'une solution ou suspension huileuse ou d'une solution ou suspension biphasique.

11. Composition selon les revendications 7 à 10, sous une forme posologique à dose unique ou sous une forme posologique à dose multiple.

12. Composition selon les revendications 7 à 11, qui est un médicament ou un produit cosmétique.

13. Composition selon les revendications 7 à 12, dans laquelle ladite au moins une bactérie probiotique est L. crispatus P17631.

14. Composition selon les revendications 7 à 12, dans laquelle ladite au moins une bactérie probiotique est L. plantarum, P17630.
